(19) Europäisches Patentamt
European Patent Office
Office européen des brevets

(11) **EP 4 494 664 A1**

(12) **EUROPEAN PATENT APPLICATION**

(43) Date of publication:
**22.01.2025 Bulletin 2025/04**

(21) Application number: **23185730.1**

(22) Date of filing: **17.07.2023**

(51) International Patent Classification (IPC):
*A61L 9/015* (2006.01)   *A61L 9/14* (2006.01)
*B60H 1/00* (2006.01)   *B60H 3/00* (2006.01)
*B60H 3/02* (2006.01)   *F24F 8/40* (2021.01)
*B03C 3/155* (2006.01)   *A61L 9/20* (2006.01)
*B60H 3/06* (2006.01)

(52) Cooperative Patent Classification (CPC):
**F24F 8/40; A61L 9/015; A61L 9/14; A61L 9/20;
B03C 3/011; B03C 3/017; B03C 3/019; B03C 3/04;
B03C 3/155; B03C 3/368; B60H 1/00742;
B60H 1/00771; B60H 1/008; B60H 1/00828;
B60H 1/00849;** (Cont.)

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(71) Applicant: **Hengst SE
48147 Münster (DE)**

(72) Inventors:
• **BORCHARD, Andreas
  48653 Coesfeld (DE)**

• **STITTERICH, Eike
  48308 Senden (DE)**
• **RÖLVER, Martin
  48329 Havixbeck (DE)**
• **PETERSDEN, Lars
  48143 Münster (DE)**
• **MENDEL, Frank
  48317 Drensteinfurt (DE)**
• **SCHOERNER, Jörg
  48612 Horstmar (DE)**
• **ROBERT, Stefan
  48356 Nordwalde (DE)**

(74) Representative: **Lohr, Jöstingmeier & Partner
Junkersstraße 3
82178 Puchheim/München (DE)**

(54) **CABIN AIR FILTER CONTROL**

(57)    A method for operating a cabin air conditioning system 1 comprising at least one of a set of n sensors $S_i$, $1 \le i \le n$, a set of $m$ air conditioning units $U\_j$, $1 \le j \le m$, a set of p air flow control elements $E_k$, $1 \le k \le p$, a human machine interface 20 (HMI), being configured to receive a set of set values $V_q$, $1 \le l \le q$ provides an improved cabin air quality if the method comprises obtaining at least one of a set of sensor values $SV_i$, $1 \le i \le n$ being indicative of an observable $O_i$ associated to the sensor $S_i$, and controlling at least one flow control element $E_k$ and/or operation of at least one of the air conditioning units $U_j$ in response to the set of sensor values $SV_i$.

Fig. 1

**EP 4 494 664 A1**

(52) Cooperative Patent Classification (CPC): (Cont.)
**B60H 3/0078; B60H 3/02; B60H 3/0625;**
A61L 2209/11; A61L 2209/111; A61L 2209/14;
A61L 2209/16; A61L 2209/212; B60H 2001/00178;
F24F 8/98; F24F 2110/50; F24F 2110/76

## Description

### Field of the invention

The invention relates to cabin air control, in particular to vehicle cabin air control and a vehicle with cabin air control.

### Description of the related art

Automobiles, with only very few exceptions, provide a cabin for persons or animals (hereinafter persons for short) being transported by use of the automobile. The same holds true for other passenger transport vehicles (boats, aircraft, trains, gondolas, etc. hereinafter vehicles, for short). The cabin of these vehicles is designed to protect the passengers in case of an impact but as well during normal operation. The cabin has to provide protection of the passengers from being exposed to weather (sun, rain, hail, snow, heat, cold) and airflow. These cabins hence provide a barrier separating the passengers from the weather outside of the cabin.

This separation, however, requires providing fresh cabin air to the cabin and to remove a corresponding air stream from the cabin. Prior to providing the fresh air to the cabin, the air is usually conditioned. This conditioning may include a number of steps. Example conditioning steps include dedusting, cooling, heating, drying, humidifying, etc.. For example, EP 0 944 941 A1 suggests determining the oxidizable portion of air being provided to an air ionizer and to increase the electrical power being provided to the air ionizer with an increase of the oxidizable portion of said air. Downstream of the air ionizer is an air humidifier and downstream of the air humidifier is an ozone-sensor. If the ozone ($O_3$) concentration in the humidified air exceeds a threshold, the voltage between the electrodes of the air ionizer is reduced to a level at which the air is still ionized but ozone is not generated.

### Summary of the Invention

The invention is based on the observation that cabin air management provides a number of challenges including a reduction of the energy consumption for air conditioning as well as to avoid health hazards of the persons being exposed to conditioned air. In particular, the reduction of the energy consumption becomes increasingly important, as the any energy being used by the cabin air conditioning system reduces the available range of battery powered vehicles and/or increases the $CO_2$ emission of fossil energy powered vehicles.

The invention is defined by the independent claim. The dependent claims relate to advantageous embodiments.

Very generally, an air conditioning system comprises at least one air inlet for sucking in air from the environment and/or from a cabin of a vehicle and or a room of a building (jointly "cabin" for short). Below, we will assume that the air conditioning system comprises an air inlet for sucking in air (outside air inlet) from a second air reservoir. The second air reservoir is mostly the environment, hence herein the environment is considered as a pars pro toto for all other second air reservoirs. Herein, air being sucked in from the second air reservoir will as well be referred to as "fresh air", even if this fresh air might be polluted by dust, ozone, nitrogen oxides, a volatile organic compound (VOC), etc. Other second air sources may include pressurized air tanks. Only for completeness, it is noted that the air in the cabin volume is considered as "first reservoir". The air conditioning system may thus further comprise at least one air inlet for sucking in air from the cabin (cabin air inlet).

Flow control means like valves (e.g., flap valves) or fans may be provided to control a first flow rate of air being sucked in from cabin and a second flow rate of air being sucked in from the environment. These flow control means may as well be used to control the airflow through the air conditioning units of the air conditioning systems and/or bypasses to these air conditioning units.

The air being sucked in is conditioned to be provided via at least one cabin air outlet to the cabin. The air conditioning system may be understood as a conduit, connecting the at least one cabin air inlet and the at least one outside air inlet via at least one air conditioning unit with at least one cabin air outlet.

These already mentioned air conditioning units are configured to condition the air flowing towards the at least one cabin air outlet by changing at least one air quality parameter. Air quality parameters are, e.g., temperature, absolute and relative air humidity, dust load, ozone concentration and the concentration of VOCs to name only a few. Air conditioning units may be sequenced and/or operated in parallel. Further, the air conditioning system may comprise one or bypass(es) for enabling one or more bypassing airflow(s), bypassing one or more air conditioning units.

Conditioned air, i.e., air that has been subjected to a treatment by at least one air conditioning unit, may be released via the cabin air outlet to a or the cabin and/or to a or the room, respectively (jointly "cabin"). Examples for air conditioning units are:

> (i) An inlet filter: The object of the inlet filter is to keep the air conditioning system downstream, of the inlet filter clear from coarse debris like leaves, stones, branches or the like, but as well to avoid that animals like mice or birds enter the portion of the air conditioning system being downstream of the initial filter. The initial filter may hence be considered as a coarse filter. Mostly, these inlet filters are sieves retaining particles being bigger than the pore size of the filter, but other principles of separation may be used as well or in addition, e.g., inertial separation. In other words, the inlet filter may comprise at least a sieving filter element and/or an inertial separator (e.g., a cyclone). Inertial separators provide the advantage that rain or snow being entering the inlet can be separated prior to providing the airstream to water sensitive other conditioning stations or even to the cabin itself. An airflow being obtained by removing cabin air may be provided only to air conditioning units system downstream of the inlet filter, as it is not to be expected that coarse debris is transported with the corresponding airstream. The cabin air inlet may hence be connected via a duct with at least one air conditioning unit located downstream of the inlet filter. Avoiding the initial filter reduces the pressure drop required for recirculating cabin air through the air conditioning system and hence contributes to reduce the energy consumption of the vehicle.

(ii) An air cooler: Air coolers have a warm air inlet (as well referred to as hot air inlet) and a cold air outlet being in fluid communication via a conduit of the air cooler. The conduit may be in thermal contact with a heat sink. For example, the conduit may be or comprised by a heat exchanger having a cold coolant inlet and a hot coolant outlet. The coolant leaving the hot coolant outlet may be cooled down again, e.g., using a heat pump or an evaporation cooler, to thereby obtain a cold coolant that may be recircled to the cold coolant inlet. In this example, the heat sink is the coolant. In another example, the heat sink may be a cold surface of a Peltier element. The cooler may be configured and/or controlled to cool warm air being received via the warm air inlet below the condensation temperature of the cooled air to thereby dry the air. In this specific example, the cold air stream leaving the cold air outlet has a lower total humidity than the warm air stream entering the hot air inlet. Air cooling as well requires a significant amount of energy.

(iii) An electrostatic precipitator: An electrostatic precipitator provides for dedusting of an air stream being received via an inlet of the electrostatic precipitator being in fluid communication with an outlet of the electrostatic precipitator. Further, electrostatic precipitators, at least reduce oxidizable components (like VOCs) in the air and may be used to produce ozone, being a disinfectant on the one hand but exposure of humans or animals to ozone should be avoided. In an electrostatic precipitator air and in particular particles being suspended in the air are first ionized and can subsequently be collected at an oppositely charged electrode. An example of an electrostatic precipitator is described in PCT/EP2023/053663 being incorporated herein by reference as if fully disclosed.

(iv) A secondary filter: The secondary filter is preferably configured to filter smaller particles being (still) suspended in the airstream. In a preferred example, the secondary filter is an electret or an active polarization filter as disclosed in, e.g., the EP-Application 22202915.9 (incorporated herein by reference as if fully disclosed). The secondary filter may as well be a or comprise an activated charcoal filter.

(v) An ozone filter: An ozone filter reduces the ozone concentration in the airflow passing the ozone filter. An example for an ozone filter is an activated charcoal filter.

(vi) A humidification device: A humidification device increases the water vapor concentration of an air stream passing the activated humidification device. Example humidification devices are scrubbers and water spray nozzles.

(vii) UV-treatment / ionization radiation treatment unit: These allow to reduce the bacterial load of the air stream passing through the air conditioning system. Further the VOC concentration may be reduced using UV-radiation.

(viii) A heater: A heater allows to increase the temperature of the air flow passing the heater. The thermal energy required to heat the air flow may be excess heat produced by the vehicle's drive system (hot engine coolant, hot battery coolant, a condenser of a heat pump, ...).

(ix) A heat exchanger: A heat exchanger has a first duct and a second duct being in thermal communication, usually via a duct wall, i.e., a heat exchange of fluids flowing the first duct and the second duct may take place, while the first and the second fluid remain separated, i.e., they do not mix. The first duct has a first inlet being connected to the cabin air inlet and a first outlet. The second duct has a second inlet and a second outlet. The second inlet may be connected (potentially via other air conditioning units) to the outside air inlet of the aid conditioning system. The second outlet may be connected (potentially via yet other air conditioning units) to at least one cabin air outlet. The heat exchanger may hence be configured to cool or heat air being sucked in from the second air source by drawing or receiving thermal energy from an air flow being removed e.g., from the cabin.

Each of these optional air conditioning units has an inlet and an outlet being in fluid communication with each other via connections (i.e., air ducts) of the respective air conditioning unit. In other words, an airflow that enters an air conditioning unit is conditioned while flowing from an air conditioning unit's inlet to the air conditioning unit's outlet, assuming the air conditioning unit to be operative. The air conditioning unit interacts with the airflow and changes at least one air quality parameter.

The inlet of the most upstream air conditioning unit is preferably connected (i.e., in fluid communication) with a system's air inlet, e.g., with the air inlet for sucking in air from the cabin (the cabin air inlet) or from a second air source, like the environment and referred to as outside air inlet. In a preferred example, the most upstream air conditioning unit can be an inlet filter. The outlet of the most upstream air conditioning unit may be in fluid communication with the subsequent downstream air conditioning unit. For example, the outlet of the initial filter may be connected to the hot air inlet of an air cooler. The cold air outlet may be connected, e.g., to an inlet of at least one of an electrostatic precipitator, a secondary filter, a heater, a humidifier, a heat exchanger or the any other air conditioning unit. One may hence summarize that at least two air conditioning units may be sequenced by a corresponding connection (an air duct).

Further, there may be one or more bypass connections (bypasses, for short) between air conditioning units. For example, an ozone filter which may be positioned downstream of an electrostatic precipitator may have a bypass allowing to provide an airflow from an outlet of an upstream (relative to the bypassed air conditioning unit) air conditioning unit to an inlet of an air conditioning unit being downstream of the bypassed air conditioning units and/or a cabin air outlet. The air conditioning system may have flow control means associated to a bypass, e.g., one or more valves and/or fans for controlling the flow rate through the associated bypass and/or the bypassed air conditioning unit.

The air conditioning system may further comprise at least one air sensor configured to measure an observable of the air and/or the airstream. Example sensors are a temperature sensor, a pressure sensor, a pressure difference sensor, a flow sensor, an anemometer, a compound concentration sensor or the like. Examples for compound concentration sensors are $O_2$-concetration sensors, $CO_2$-concentration sensors, Methane ($CH_4$) concentration sensors, oxidizable portion concentration sensors, to name only a few. Observables characterizing physical or chemical properties of the air flow can be used to describe the air quality. These are thus as well referred to as air quality parameters as already explained above.

Further, the air conditioning system may comprise at least one fan configured to provide an airflow from the system's air inlet via the air conditioning system to the system's air outlet. The air conditioning system may further comprise valves configured to control an airflow between defined sections of the air conditioning system. Herein these are generalized under the superordinate term of flow control element. For example, an air conditioning system may comprise two (or more) air inlet openings. One inlet opening may be open to the environment and the other may be open to the cabin of a vehicle or to a room of a house. A flap or any other kind of valve(s) may be used to adjust the relative airflows of the corresponding stream to the same air outlet(s) or to different outlets of the air conditioning system. The same effect can be obtained by the use of fans for different branches of the air conditioning systems.

The air conditioning system may further comprise at least one controller. The controller may be connected by at least one signal line to at least one of the sensors identified herein. The controller is hence configured to obtain sensors signals and to process sensors signals.

The controller may further be connected by at least one control line, e.g., via a corresponding driver unit, to at least one air conditioning unit. The controller may as well be configured to control operation of the control unit, e.g., control the thermal energy being removed from an airstream in a cooler and/or the voltage at the electrodes of an electrostatic precipitator and/or the voltage at the electrodes of an active polarization filter and/or operation of an air humidifier and/or control the temperature at a hot air outlet of a heater. The controller may as well be configured to read or measure system parameters of air conditioning units like, e.g., an electrical power being drawn by any air conditioning unit, a temperature or a pressure drop between the air conditioning unit's inlet and outlet etc. In short, one may assume that the controller is configured to obtain any available data from any air conditioning unit.

In addition, the controller may be configured to exchange data with a power train control module or that the power train control module is the controller. This provides the ability to control operation of the air conditioning system based on other parameters of the vehicle as will be explained below in more detail. These other parameters can as well be considered as sensor values being indicative of an observable, regardless if these have been measured directly or indirectly.

The controller is preferably configured to control operation of the fan(s) and/or the valve(s) and the air conditioning unit(s) based on data being obtained from said at least one sensor.

The invention provides a method for improving cabin air quality, operational safety and for reducing the energy consumption associated to operation of the air conditioning system.

Generalizing, the cabin air conditioning system comprises at least one of a set of $n$ sensors $S_i$, $1 \leq i \leq n$, a set of $m$ air conditioning units $U_j$, $1 \leq j \leq m$, a set of $p$ air flow control elements $E_k$, $1 \leq k \leq p$ and at least one human machine interface (HMI), The HMI may be configured to receive a set of set values $V_q$, $1 \leq l \leq q$. In an aspect, the invention comprises at least one of the steps: (i) obtaining a sensor value $SV_i$, $1 \leq i \leq n$ being indicative of an observable $O_i$ associated to the sensor $S_i$ and (b) controlling at least one flow control element $E_k$ and/or operation of at least one of the air conditioning units $U_j$ in response to the at least one sensor value $SV_i$. Preferably, the step of controlling may aim for a cabin air conditioning that meets the at least one set value $V_q$ within an error margin.

Preferred set values are temperatures $\vartheta$ (e.g., of different portions of the cabin), relative air humidity $\varphi$ and/or an air flow rate through a cabin air outlet or a (sub)set of cabin air outlets.

In a preferred example, the method may comprise determining the number of occupants in the cabin and if the number of occupants is zero, opening a bypass to an ozone-filter being downstream of an electrostatic precipitator, controlling the electrostatic precipitator to thereby produce ozone and provide a flow of ozone enriched air from the electrostatic precipitator to a cabin air outlet. This measure allows to clean the cabin from odors that remained after use in the cabin. Further, the surfaces in the cabin are subjected to a disinfection (at least partially) due to the contact with the ozone enriched air. In addition or alternatively, the ozone enriched air may as well be circulated through other components of the air conditioning system, e.g., through an initial filter, a secondary filter and/or humidifiers and/or air coolers and/or air dehumidifiers and/or connections ducts or other air ducts. This measure reduces the bacterial contamination of the respective component, and hence reduces health risks associated with the operation of air conditioning systems. In a typical example, a passenger vehicle would be used by a number of passengers for transporting these passengers in a cabin of the vehicle from a departure location to a destination. The air conditioning system may operate during the transport to provide the passengers with conditioned air. Once the transfer is completed and the passengers debarked, ozone enriched air provided by the electrostatic precipitator or another ozone source can be provided to the cabin and/or components of the cabin air conditioning system to thereby remove remaining odors and/or to reduce bacterial contamination. The process may be started manually, in particular from a remote device, e.g. a cell phone. In a preferred example, the step of providing ozone enriched air to the cabin is performed in response to sensor values. For example, if a VOC-sensor shows a VOC concentration above a threshold, the step of providing ozone enriched air to the cabin is performed. In another example, the step of providing ozone enriched air to the cabin may be performed in response to seat occupancy detection sensor signals. For example, the step of providing ozone enriched air may be performed if a given number or "person hours" exceed a threshold. The term *person hours* shall describe the usage of the cabin by persons. If a single person uses the cabin for an hour this corresponds to one "person hour". If during the same one-hour trip four persons occupied the cabin this corresponds to four person hours.

The number of persons being in the cabin can be determined based on seat occupation sensors and/or camera devices and/or thermal power provided by the persons in the cabin, etc.. The time a person uses a seat can be measured using a simple clock, the latter is typically integrated in any electronic controller.

The method is preferably executed by or using a controller executing program instruction implementing the method steps. Detecting the number of persons in the cabin can be performed by evaluating sensor signals from safety belt lock recognition sensors, being present in (almost) any modern car. For example, if all safety belt lock recognition sensors provide a signal being indicative of the information *"safety belt lock open"*, this may be considered as a detection that no person is present. If a number of safety belt lock recognition sensors provide a signal indicative of the information *"safety belt lock closed"*, the number can be considered to correspond to the number of persons in the cabin. Alternatively or in addition, at least one IR sensor and/or at least one seat-occupied sensor mat may be used and/or a camera with image recognition system can be used to detect the number of persons in the cabin. These sensors are often already present in modern passenger vehicles (e.g., for controlling airbag activation or as part of an intrusion alarm system), but such sensor values have not yet been used to control and hence operate cabin air conditioning systems. In a preferred embodiment, the controller of the cabin air conditioning is hence either integrated in a central vehicle control system, e.g., in a power train control module, or may have at least one data exchange port configured to exchange date with a central vehicle control system, e.g., a power train control module. Thereby, not only access to almost any sensor signal of the vehicle is possible, but the as well weight and cost savings can be obtained, by avoiding providing redundant sensors. For example, modern combustion engines have an outside air temperature sensor and/or an air pressure sensor and/or an air humidity sensor to determine based on the corresponding sensor data the oxygen flow into the combustion chamber and hence the required fuel quantity to be added to the air and or the ignition timing. This corresponding sensor value can as well be used by the air conditioning system as a representative of the ambient air temperature. Further, a sensor signal of a rain sensor for wiper control can be used to obtain information on the relative air humidity of the ambient air.

Going back to the example of detecting the presence of persons or animals in the cabin: In a preferred example, detecting that the number of persons is zero may be obtained by measuring the $CO_2$ concentration of the cabin air and/or the $O_2$ concentration of the cabin air. A change in the water concentration of the cabin air (air humidity measurement) may as well be used to detect the presence of persons. If these concentrations remain constant although no air exchange takes place, no person is present in the cabin. As already apparent, measuring a concentration shall express that a sensor value being indicative for the respective concentration is obtained from a corresponding sensor.

In other words, the method may comprise determining if the number of occupants in the cabin is zero or not. This determining may be, e.g., based on at least one sensor value as explained above. If the number of occupants is zero, the method may proceed to the step of operating a or the, respectively, electrostatic precipitator to thereby produce ozone and to provide a flow of ozone enriched air from the electrostatic precipitator to another air conditioning unit (e.g. through an air filter) without subjecting the ozone enriched airflow to an ozone filter prior to providing it to the at least one another air conditioning unit and/or the cabin air outlet.

In a preferred example, the method may further comprise stopping to provide the flow of ozone enriched air from the electrostatic precipitator to the cabin air outlet without subjecting the ozone enriched air to an ozone filtration or another ozone removal process (herein to be included in the term ozone filtration). Stopping may be triggered by at least one of the following events:

(i) The ozone concentration in the cabin exceeds a threshold,

(ii) presence of persons is detected in the cabin (including an attempt to open a cabin door), and/or

(iii) a preset time interval lapsed.

In the first event premature degradation of the material inside the cabin due to excessive ozone in the cabin air is reduced. In the second event, harm to the persons due to being exposed to ozone is reduced, if not fully avoided. In the third event, premature degradation of the material is reduced, and energy consummation is reduced, and the cabin can be prepared to be occupied as explained below in more detail.

In a preferred example, the method comprises locking the cabin doors against being opened from the outside during the step of providing a flow of ozone enriched air from the electrostatic precipitator to the cabin air outlet preferably via a bypass that bypasses an ozone filter. This measure ensures that no person can enter the cabin while the ozone treatment is in process. In a preferred example, the cabin comprises an emergency unlocking system, enabling to unlock the cabin doors even if the ozone concentration in the cabin is or can be expected to be above an ozone concentration threshold, i.e. if the doors are locked during step of providing a flow of ozone enriched air from the electrostatic precipitator to the cabin air outlet (usually via a bypass that bypasses an ozone filter).

The method may further comprise flushing the cabin with air having a low ozone concentration while removing ozone enriched air from the cabin after stopping the step of providing a flow of ozone enriched air from the electrostatic precipitator to the cabin air outlet. In a preferred example, the cabin doors are kept locked during the step of flushing while removing and the cabin doors are unlocked after a preset amount of air having a low ozone concentration has been flushed into the cabin and/or if the ozone concentration in the cabin air is below an ozone concentration threshold, preferably unless a person or animal inside the cabin has been detected.

In a preferred example, the method may comprise, after stopping the flow of ozone enriched air from the electrostatic precipitator to the cabin air outlet, the step of circulating cabin air through the ozone filter. Unless persons are detected the step of circulating cabin air through the ozone filter may be performed with a delay of at least $t$ $min$ (minutes), wherein $t \in \{1, 2, 3, 4, 5, 10, 20, 30, 45, 60\}$ after the step of stopping the flow of ozone enriched air from the electrostatic precipitator to the cabin air outlet. The delay can be considered as a dwell time. The dwell time may be preset to a first dwell time value and may be increased (or decreased) to a second dwell time value by a user of the cabin via the HMI. The first dwell time value may depend on other parameters, like, e.g., the location and/or the time at which the vehicle was parked. The step of circulating has the advantage that the clean air being inside the cabin remains in the cabin (is recircled) and no energy is needed to clean air from the second reservoir replacing the ozone enriched air inside the cabin.

At least during normal operation of the air conditioning system, the method may comprise removing a first air stream from the cabin and providing at least a portion of it at a first flowrate to a cabin air inlet of the cabin air conditioning system. This first air stream may hence be (re)conditioned in the cabin air conditioning system and may be provided after being conditioned to at least one cabin air outlet of the air conditioning system.

The method may further comprise removing a second air stream from another air source. This another air source may be, e.g., the environment the cabin is exposed to or in other words the ambient air. At least a portion of the second air stream by be provided at a second flow rate to a or the cabin air outlet of the cabin air conditioning system via at least one of the air conditioning units.

The total flow rate being provided to the cabin air outlet(s), may hence be the sum of the first flow rate and the second flow rate. The total flow rate, the first flow rate and the second flow rate may hence be varied to thereby control at least one cabin air parameter, e.g., to meet at least one set value within a given error margin. In a preferred example, the first flow rate and the second flow rate are determined by minimizing the energy consumption of the air conditioning unit as a function of the first flow rate and the second flow rate, cabin air parameters and second air source parameters and at least one air quality parameter threshold value. For example, the method may comprise determining the first flow rate and the second flow rate as a function of at least one of the number of passengers in the cabin, at least one first sensor value $SV_c$ being indicative of cabin air quality, at least one second sensor value $SV_o$ being indicative of the quality of the second portion of air, the energy consumption of the air conditioning system to maintain a given set of observables being indicative of cabin air quality within respective limits and/or information about the travelling route of the cabin. This information contributes to determining the lowest total flow rate that is required to meet at least one set value within a given error margin. Hence, fan power can be reduced contributing to a reduction of the energy consumption of the air conditioning unit. For example, if a set temperature is below the cabin air temperature, the method may comprise determining if the cabin air temperature is lower than the ambient temperature and control the air flow control elements to minimize the flow rate of the air stream that has the higher inlet temperature. Thereby the power consumption of the cooler is minimized. In an example, one may assume a cabin has been parked in a sunny environment. The cabin air temperature has been heated up, due to the exposure of the sun radiation above the ambient temperature. In this case the method may comprise minimizing the first flow rate while maximizing the second flow rate. Once the cabin air has been cooled down below the temperature of the ambient air (as an example for a second air source), the method may comprise minimizing the second airflow and maximizing the first airflow instead. Said minimizing and maximizing may be limited by other constraints. For example, at least if persons are present in the cabin, the second flow rate preferably is set at or above a minimum to ensure the $O_2$ concentration does not drop below a $O_2$ threshold and/or the $CO_2$ concentration does not exceed a $CO_2$ threshold.

If the first flow or the second flow rate is maximized, it is preferably maximized under the constraint of a given total flow rate. In other words, said maximizing may include balancing the total energy consumption of the system. This is favorable to reduce the total energy consumption of the air conditioning system, for example if the second flow rate is maximized and the dust load of the second air source is higher than the dust load of the cabin air as in this case the power consumption of an electrostatic precipitator for dedusting the second air flow may increase if the second flow rate increases.

The method may further comprise determining a minimum mean second flowrate that is required to maintain the oxygen concentration in the cabin above a given oxygen concentration threshold, wherein determining the minimum mean second flowrate is based on the number of passengers in the cabin and/or based on at least one sensor value being indicative for the oxygen and/or $CO_2$ content in the cabin, and/or based on a calculated expected trip time and/or based on the maximum passenger capacity of the cabin and/or a change in the $O_2$ concentration in the cabin air as a function of the second flow rate and/or a change in the $CO_2$ concentration in the cabin air as a function of the second flow rate.

The method may further comprise controlling the flow control elements to provide a mean second flow rate at or above the determined minimum mean second flow rate, wherein the mean is calculated over a given time. This measure enables to ensure that the $O_2$ concentration does not drop below an $O_2$ threshold and/or

the $CO_2$ concentration does not exceed a $CO_2$ threshold. It is sufficient if the minimum mean second flow rate is maintained as passenger cabins usually have enough volume to ensure that no physical harm is caused to the passengers in case no fresh (=oxygen rich) air is supplied to the cabin for a limited time. Thus, the second flow rate may drop below the minimum mean second flow rate. For example, if one or more senor values indicate that ambient air quality is below a corresponding threshold (to be observed e.g., in tunnels, in the vicinity of freshly fertilized fields, ...) the second flow rate may be reduced below the calculated minimum mean second flow rate. Preferably, the second flow rate is increased at least to the minimum mean second flow rate if the one or more senor values indicate that ambient air quality is above a corresponding threshold and/or if the $O_2$ concentration in the cabin air drops below a $O_2$ threshold and/or the $CO_2$ concentration exceeds a $CO_2$ threshold. In a preferred example the second flow rate is increased above the determined minimum mean second flow rate at least until mean second flow rate is equal to the minimum mean second flow rate and/or the $O_2$ concentration in the cabin is at or above an $O_2$ threshold and/or the $CO_2$ concentration is at or below a $CO_2$ threshold. An increase of the second flow rate may as well be triggered based on trip data.

In an example the method my comprise scanning a planned route for sections in which unfavorable outside air parameters can be expected (e.g. in tunnels, sections in the vicinity of industrial complexes, ...) and increasing the second flow rate in a section prior the section in which unfavorable outside air parameters can be expected and/or after the which unfavorable outside air parameters can be expected have been passed to a value above the determined minimum mean second flow rate while decreasing the second flow rate below determined minimum mean second flow rate while passing the sections in which unfavorable outside air parameters can be expected and/or are determined. In a preferred example, the mean second flow rate is maintained at or above the minimum mean second flow rate.

In yet another example, the second flow rate is reduced below the determined minimum mean second flowrate if it can be expected that a destination in reached within a small remaining trip time. Example values for a small remaining trip time can be 30s, 1min, 1.5min, 2min, 2.5min, 3min, 4 min, 5 min or even longer time intervals. At the end of the trip, it can be expected that a cabin door will be opened which will provide an uncontrolled huge air exchange between the cabin air and the outside air and a reduced $O_2$-concentration in the cabin will be increased 'automatically' due to the uncontrolled huge air exchange. The method hence allows to reduce the energy consumption required to condition outside air prior to providing it to the cabin air outlet, being in most cases higher than the energy consumption to condition recircled cabin air.

Further, the method may comprise determining a minimum total flowrate of the total air streams being provided to the cabin for maintaining at least one air quality parameter above or below a threshold, wherein the air quality parameter is different from the oxygen concentration of the cabin air. Examples of these other air quality parameters are for example the $CH_4$ concentration in the cabin air, relative humidity of the cabin air, the concentration of oxidizable components of the cabin air (e.g., VOCs) and the like. Generalizing, the other air quality parameters can be any cabin air quality parameter that can be improved by at least one air conditioning unit and hence does not necessarily require an increase of the second flow to be improved. For example, concentration of oxidizable components of the cabin air can be reduced using an electrostatic precipitator and/or an activated charcoal filter. The relative humidity may be controlled by dehumidifiers, coolers, and/or humidifiers.

The method may further comprise determining a first flow rate as a function of at least the minimum total flow rate and the minimum second flow rate.

The method may further comprise controlling at least one of the air flow control elements $E_k$ to meet at least one of the following conditions: the minimum second flowrate provided to the cabin and the minimum first flow rate is provided to the cabin.

In an example, the method may comprise receiving the at least one second sensor value $SV_o$ from an engine manifold intake temperature sensor and/or engine manifold intake humidity sensor.

In an example, the air conditioning system is included in a passenger vehicle. Example sensors that may be comprised in the set of sensors are an engine manifold intake air temperature sensor, an engine manifold intake air humidity sensor, a battery coolant inlet temperature sensor, a battery coolant outlet temperature sensor, a seatbelt lock recognition sensor, a seat occupation sensor, a rain sensor, a scattered light sensor, a light sensor, a GPS-sensor (as a pars pro toto for a position sensor) and a dew point sensor. Each of these sensors may or may not be comprised in the set of sensors.

In a preferred example, the method may comprises identifying for a set $S$ of air conditioning units a power $P_S(q_1,q_2)$ being the sum of the power consumptions of each member of the set for a change of an observable being changed by operation of the respective air conditioning unit at a given airflow through the respective air conditioning unit and optionally of at least one fan of the air conditioning system to provide said given airflow through the air conditioning units of the set. The method may further comprise determining a first flow rate $q_1$ and a second flow rate $q_2$ to meet the condition $\nabla P(q_1,q_2) = \vec{0}$ and to control the first and the second flow rate by setting the first flow rate to $q_1$ and the second flow rate to $q_2$. In a preferred example it is further determined if the Determinant of the Hesse Matrix $H(P(q_1, q_2))$ is positive definite ($det\ H(q_1, q_2) > 0$) and then the method proceeds to control the first flow rate and the second flow rate by setting these to the tuple $(q_1, q_2)$ having the lowest value

for $P(q_1,\ q_2)$ and $det\ H(q_1,\ q_2) > 0$ and $\nabla P(q_1, q_2) = \vec{0}$. Thereby, the power consumption for changing the observables can be minimized. In other words, in a preferred example, the first flow rate and the second flow are preferably set to values $q_1$ and $q_2$, respectively, wherein the values $q_1$ and $q_2$ are selected (in no matter way) such that $\nabla P(q_1, q_2) = \vec{0}$ and preferably as well that $det\ H(q_1, q_2) > 0$.

## Description of the Figures

In the following, the invention will be described by way of example, without limitation of the general inventive concept, on examples of embodiment with reference to the drawings.

Fig. 1 shows an air conditioning system.

Fig. 2 shows a flow diagram of a method for controlling an air conditioning system.

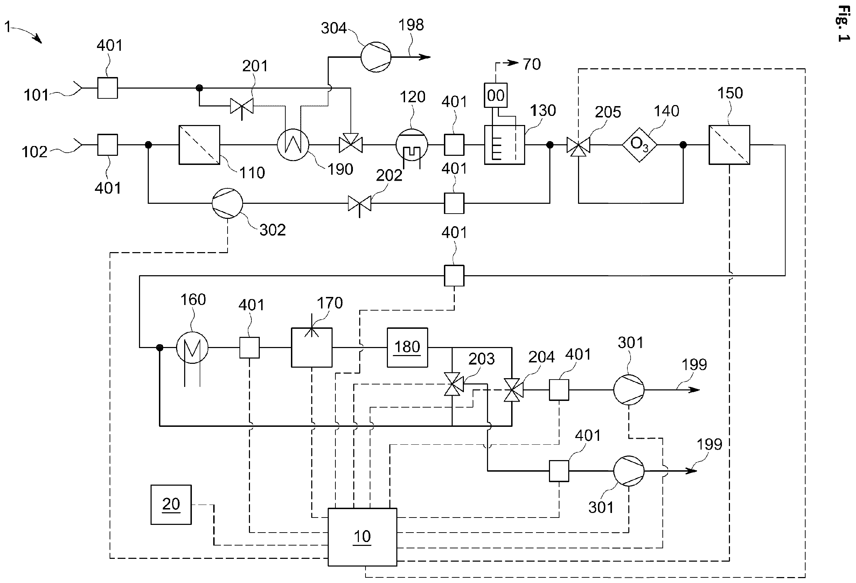

In FIG. 1 an air conditioning system 1 is shown. The air conditioning system 1 may be used as and hence be a passenger vehicle air conditioning system. The air conditioning system 1 comprises at least one cabin air inlet 101 and/or a secondary air source inlet 102 ("outside air inlet"). These may be connected via a number of ducts (indicated as connecting lines) to at least one cabin air outlet 199.

These ducts may comprise and/or connect a number of air conditioning units. Example air conditioning units are and initial filter 110, an air cooler 120, an electrostatic precipitator 130, an ozone filter 140, a secondary filter 150, a heater 160, a humidifier 170, an aerosol dispenser 180 and a heat exchanger 190. Each of these air conditioning units is optional, but a preferred example at least two of these are present.

The air conditioning system may further comprise a number of valves 201 to 205 and/or fans 301, 302 to control the airflow through the system.

In a preferred example, cabin air is withdrawn from the cabin of a passenger vehicle via at least one cabin air inlet 101. A sensor unit 401 may be configured to provide at least one sensor value being indicative of at least one observable like cabin air temperature, $O_2$-concentration, $CH_4$-concentration, $CO_2$-concentration, etc.. The at least one sensor unit 401 is preferably connected by a data link to a controller 10 being configured to obtain the sensor value(s) from the at least one sensor unit. In FIG. 1, the data links between the at least one sensor unit 401 and the controller 10 are indicated as dashed lines and some of these have been partially omitted to declutter the figure. It may be assumed that any sensor unit 401 may be connected by a data link to at least one controller. These data links can be realized by cables (electrical, optical, ...) or as well by wireless data link connections.

Fans, e.g., fans 301, 302, 303, 304 and valves, e.g., valves 201 to 105 may be connected to the controller 10 by control lines, which are as well indicated as dashed lines, some of which have been omitted to declutter the figure. It may be assumed that any fan 30x and/or valve 20x and/or any aur conditioning unit 1x0 may be connected by a data link and or a control link to the controller 10. The controller 10 may hence be configured to control operation of any of these devices and or obtain data from these. Example date include but are not limited to valve statuses, inlet or outlet temperatures, pressures, flow rates, etc..

As can be seen, the system preferably has at least one cabin air inlet 101 and at least an outside air inlet 102, at least one cabin air outlet 199 and at least one outside air outlet 198. In FIG. 1 two cabin air outlets 199 are depicted, but the number is usually higher. During operation, at least a portion of the air being sucked out of the cabin via at least one cabin air inlet 101 may be replaced by air being sucked in via the at least one outside air inlet 102, e.g. to thereby remove products of the passenger's metabolism(s) (e.g. $CH_4$, $CO_2$) from the cabin and replenish air components (e.g. $O_2$) being required by the passengers. The replaced portion of the air may be provided to the environment via the outside air outlet 198.

As shown in FIG. 1, the excess air being provided from a cabin air inlet to an outside air outlet 198 may be routed via an optional heat exchanger 190. The air entering the air conditioning system 1 via the at least one outside air inlet maybe provided to a second inlet 192 of the heat exchanger 190, be warmed or cooled down in the heat exchanger 190 and leave the heat exchanger 190 via a second outlet 194. In the optional heat exchanger 190, the temperature of the outside air is (pre)conditioned to or at least towards the temperature of the cabin air. In a preferred example, the heat exchanger 190 may comprise an optional bypass duct, to configured to suck in fresh air without cooling or heating it using the heat exchanger. Routing of the air through the bypass or the heat exchanger may be controlled by the controller 10 controlling corresponding flow control means.

Downstream of the optional heat exchanger, the remaining portion of the airflow being removed from the cabin and the portion that has been sucked in via the outside air inlet may be mixed. In a downstream air conditioning unit, the air stream may be cooled (see optional cooler 120). Cooling may as well result in dehumidification. As well downstream, the air may be subjected to an optional electrostatic precipitator 130. Further downstream the air flow may be subjected to an optional ozone filter 140 and/or an optional secondary filter 150. The optional secondary filter 150 may be or comprise a "sieving" filter and/or an electret filter and/or an active polarization media filter. Downstream of the cooler, the air conditioning system may comprise at least one heater 160 configured to heat the air flow up, if required. Further, an optional humidifier 170 and/or an optional aerosol dispenser 180 may be in the flow path of the airflow towards at least one cabin air outlet 201.

As already apparent, the fans 301 to 304 and the valves 201 to 205 are preferably controlled by the optional controller 10. The controller 10 may be configured to implement a method as described herein, e.g., the method of one of the claims.

As can be seen in FIG. 1, the air conditioning system 1 is preferably configured to remove a first air stream from the cabin via the cabin air inlet 101 and to provide at least a portion of it at a first flowrate $q_1$ to a cabin air outlet 199 of the cabin air conditioning system 1. A second air stream from another air source and may be drawn via the second air inlet 102 (the outside air inlet) and at least a portion of the second air stream may be provided at a second flow rate $q_2$ to the cabin air outlet 199 of the cabin air conditioning system 1. The first flow rate $q_1$ and the second flow $q\_2$ rate may be determined according to a method as disclosed herein. The method may comprise determining the number of passengers in the cabin and/or at least one first sensor value $SV_c$ being indicative of cabin air quality, at least one second sensor value $SV_o$ being indicative of the quality of the second portion of air and/or the energy consumption of the air conditioning system to maintain a given set of observables being indicative of cabin air quality within respective limits and/or information about the travelling route of the cabin.

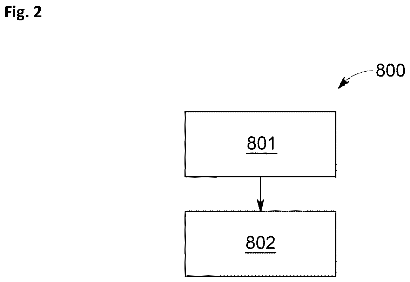

FIG. 2 depicts a method 800. In general, the method may comprise the method step 801 of obtaining at least one of a set of sensor values $SV_i$, $1 \leq i \leq n$ being indicative of an observable $O_i$ associated to a sensor $S_i$. The sensors $S_i$ may be integrated in or form a sensor unit 401 in FIG. 1.

The method may further comprise the step 802 of controlling at least one flow control element $E_k$ and/or operation of at least one of the air conditioning units $U_j$ in response to the set of sensor values $SV_i$. The method steps 801, 802 may be executed by the optional controller 10 (see FIG. 10). The controller 10 may comprise and/or be connected to an optional memory 11 comprising program instructions for executing the method steps as disclosed herein.

In an example, the method may comprise, based on a sensor value, determining if the number of occupants in the cabin is zero, and if the number is zero as part of method step 802: opening a bypass to an ozone-filter being downstream of an electrostatic precipitator, operating the electrostatic precipitator to thereby produce ozone and providing a flow of ozone enriched air from the electrostatic precipitator to a cabin air outlet.

In another example, if the number of occupants in the cabin is zero, the method may comprise as part of method step 802: operating the electrostatic precipitator 130 to thereby produce ozone and provide a flow of ozone enriched air from the electrostatic precipitator 130 to another air conditioning unit without subjecting the ozone enriched airflow to an ozone filter located in the flow path between the electrostatic precipitator and another air conditioning unit. In the example of FIG. 1, these another air conditioning units are, e.g., the initial filter 110 and the heater 160. From both, the flow of ozone enriched air may be provided to the at least one cabin air inlet 199.

Further, the method may comprise monitoring the ozone concentration in the cabin and/or the duration the flow of ozone enriched air from the electrostatic precipitator to the cabin air outlet 199 and, during the step of providing said flow, monitoring sensor values indicative of observables that may be considered as indication persons inside the cabin. In case the ozone concentration in the cabin exceeds a threshold and/or presence of humans or animals is detected and/or if a preset time interval lapsed, the methods step 802 may comprise stopping the flow of ozone enriched air from the electrostatic precipitator 130 to the cabin air outlet 199. Subsequently, cabin air may be circulated through the ozone filter to thereby remove the ozone from the cabin air (as a part of step 802).

**List of reference numerals**

| | |
|---|---|
| 10 | controller (optional) |
| 20 | HMI (optional) |
| 101 | cabin air inlet (optional) |
| 102 | secondary air source inlet / outside air inlet (optional) |
| 110 | initial filter (optional) |
| 120 | cooler (optional) |
| 130 | electrostatic precipitator (optional) |
| 140 | ozone filter (optional) |
| 150 | secondary filter (optional), e.g., active polarization filter |
| 160 | heater (optional) |
| 170 | humidifier (optional) |
| 180 | aerosol dispenser (optional) |
| 190 | heat exchanger (optional) |
| 198 | outside air outlet (optional) |
| 199 | cabin air outlet (optional) |
| 201 | inlet valve (optional) |
| 202 | inlet valve (optional) |
| 210 | bypass valve (optional) |
| | |
| 301 | first example fan (optional) |
| 302 | second example fan (optional) |
| 303 | third example fan (optional) |
| 304 | fourth example fan (optional) |
| | |
| 401 | sensor unit (optional) |

**Claims**

1. A method for operating a cabin air conditioning system (1), wherein the cabin air conditioning system (1) comprises at least one of:

- a set of $n$ sensors $S_i$, $1 \leq i \leq n$
- a set of $m$ air conditioning units $U_j$, $1 \leq j \leq m$,
- a set of $p$ air flow control elements $E_k$, $1 \leq k \leq p$,
- a human machine interface HMI, being configured to receive a set of set values $V_q$, $1 \leq l \leq q$

**characterized in that** the method comprises:

(i) obtaining at least one of a set of sensor values $SV_i$, $1 \leq i \leq n$ being indicative of an observable $O_i$ associated to the sensor $S_i$,
(ii) controlling at least one flow control element $E_k$ and/or operation of at least one of the air conditioning units $U_j$ in response to the set of sensor values $SV_i$.

2. The method of claim 1, **characterized in, that** the method comprises determining if the number of occupants in the cabin is zero, and if the number is zero: opening a bypass to an ozone-filter (140) being downstream of an electrostatic precipitator (130), operating the electrostatic precipitator (130) to thereby produce ozone (140) and providing a flow of ozone enriched air from the electrostatic precipitator (130) to a cabin air outlet (199).

3. The method of claim 1 or 2, **characterized in, that** the method comprises determining if the number of occupants in the cabin is zero and if the number of occupants is zero, operating a or the, respectively, electrostatic precipitator (130) to thereby produce ozone and provide a flow of ozone enriched air from the electrostatic precipitator (130) to another air conditioning unit without subjecting the ozone enriched airflow to an ozone filter located in the flow path between the electrostatic precipitator (130) and another air conditioning unit.

4. The method of claim 2 or 3, **characterized in that** it further comprises stopping to provide the flow of ozone enriched air from the electrostatic precipitator (130) to the cabin air outlet (199), if at least one of the following conditions is met:

- an ozone concentration in the cabin exceeds a threshold,
- presence of humans or animals is detected,
- a preset time interval lapsed.

5. The method of claim 4, **characterized in that** it further comprises after stopping the flow of ozone enriched air from the electrostatic precipitator (130) to the cabin air outlet: circulating cabin air through the ozone filter (140).

6. The method of one of the previous claims, **characterized in that** it comprises:

a. removing a first air flow from the cabin and providing at least a portion of it at a first flowrate to a cabin air outlet (199) of a cabin air conditioning system (1),

b. removing a second air flow from another air source and providing at least a portion of it at a second flow rate to a cabin air outlet (199) of the cabin air conditioning system (1),

c. determining the first flow rate and the second flow rate as a function of at least one of:

- the number of passengers in the cabin,
- at least one first sensor value $SV_c$ being indicative of cabin air quality,
- at least one second sensor value $SV_o$ being indicative of the quality of the portion of the second air flow,
- the energy consumption of the air conditioning system (1) to maintain a given set of observables being indicative of cabin air quality within respective limits,
- information about the travelling route of the cabin.

7. The method of claim 6, **characterized in that** the method comprises:

(i) calculating a minimum second flowrate that is required to maintain the oxygen concentration in the cabin above a given oxygen concentration threshold, wherein calculating the minimum second flowrate is based on the number of passengers in the cabin and/or based on at least one sensor value being indicative for the oxygen content in the cabin, and/or based on a calculated expected trip time.

(ii) calculating a minimum total flowrate of the total air streams being provided to the cabin for maintaining at least one air quality parameter above or below a threshold, wherein the air quality parameter is different from the oxygen concentration of the cabin air.

8. The method of claim 6, **characterized in that** the method comprises determining a minimum first flow rate as a function of at least the minimum total flow rate and the minimum second flow rate.

9. The method of claim 6, **characterized in that** the method comprises controlling the air flow control elements $E_k$ to meet at least one of the following conditions:

(i) the minimum second flowrate provided to the cabin,
(ii) the minim first flow rate is provided to the cabin.

10. The method of one of claims 2 to 9, **characterized in that** the at least one second sensor value $SV_o$ is an engine manifold intake temperature and/or humidity sensor.

11. A cabin air conditioning system (1) for a passenger vehicle, comprising at least one of:

- a set of $n$ sensors $S_i$, $1 \leq i \leq n$
- a set of $m$ air conditioning units $U_j$, $1 \leq j \leq m$,
- a set of $p$ air flow control elements $E_k$, $1 \leq k \leq p$,
- a human machine interface HMI, being configured to receive a set of set values $V_q$, $1 \leq l \leq q$

**characterized in that** the air conditioning system (1) further comprises controller (10) being configured to execute a method according to one of the previous claims.

12. The cabin air conditioning system (1) of the previous claim, **characterized in that** the controller (10) has an interface configured for exchanging data with a power train control module or that the power train control module is the controller (10).

13. A passenger vehicle, **characterized in that** is comprises a cabin air conditioning system (1) of one of the previous claims.

14. The subject matter of one of the previous claims, **characterized in that** the set of sensors comprises at least one of the following sensors:

- an engine manifold intake air temperature sensor,
- an engine manifold intake air humidity sensor,
- a battery coolant inlet temperature sensor,
- a battery coolant outlet temperature sensor,
- a seatbelt lock recognition sensor,
- a seat occupation sensor,
- a rain sensor,
- a scattered light sensor,
- a light sensor,
- a dew point sensor.

**Fig. 1**

**Fig. 2**

800

801

802

Europäisches
Patentamt

European
Patent Office

Office européen
des brevets

# EUROPEAN SEARCH REPORT

**Application Number**

EP 23 18 5730

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | JP 2019 209827 A (DENSO CORP) 12 December 2019 (2019-12-12) | 1,3-6, 10-14 | INV.<br>A61L9/015 |
| Y | * paragraph [0004] - paragraph [0147]; claims; figures * | 2 | A61L9/14<br>B60H1/00<br>B60H3/00 |
| X | US 2015/032266 A1 (WEAST JOHN C [US] ET AL) 29 January 2015 (2015-01-29) * paragraph [0017] - paragraph [0053]; claims; figures * | 1,6,11, 13 | B60H3/02<br>F24F8/40<br>B03C3/155<br>A61L9/20<br>B60H3/06 |
| X | CN 108 162 717 B (UNIV NORTHEASTERN) 17 April 2020 (2020-04-17) * the whole document * | 1,6,11, 13 | |
| Y | US 2005/169821 A1 (BOSCHERT BJORN [DE] ET AL) 4 August 2005 (2005-08-04) * paragraph [0060] - paragraph [0068]; figures 4-8 * | 2 | |
| A | JP H05 178080 A (ZEXEL CORP) 20 July 1993 (1993-07-20) * paragraph [0008] - paragraph [0014]; figures * | 1-14 | |

**TECHNICAL FIELDS SEARCHED (IPC)**

A61L
B60H
F24F
B03C
B01D

The present search report has been drawn up for all claims

| Place of search | Date of completion of the search | Examiner |
|---|---|---|
| Munich | 31 January 2024 | Endrizzi, Silvio |

EPO FORM 1503 03.82 (P04C01)

## ANNEX TO THE EUROPEAN SEARCH REPORT
## ON EUROPEAN PATENT APPLICATION NO.

EP 23 18 5730

This annex lists the patent family members relating to the patent documents cited in the above-mentioned European search report.
The members are as contained in the European Patent Office EDP file on
The European Patent Office is in no way liable for these particulars which are merely given for the purpose of information.

31-01-2024

| Patent document cited in search report | | Publication date | Patent family member(s) | | Publication date |
|---|---|---|---|---|---|
| JP 2019209827 | A | 12-12-2019 | NONE | | |
| US 2015032266 | A1 | 29-01-2015 | CN | 105339198 A | 17-02-2016 |
| | | | EP | 3024676 A1 | 01-06-2016 |
| | | | JP | 2016521655 A | 25-07-2016 |
| | | | KR | 20160003239 A | 08-01-2016 |
| | | | US | 2015032266 A1 | 29-01-2015 |
| | | | WO | 2015012826 A1 | 29-01-2015 |
| CN 108162717 | B | 17-04-2020 | NONE | | |
| US 2005169821 | A1 | 04-08-2005 | AU | 2003255629 A1 | 08-10-2003 |
| | | | DE | 10213195 A1 | 16-10-2003 |
| | | | EP | 1658186 A1 | 24-05-2006 |
| | | | US | 2005169821 A1 | 04-08-2005 |
| | | | WO | 03080375 A1 | 02-10-2003 |
| JP H05178080 | A | 20-07-1993 | NONE | | |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 0944941 A1
- EP 2023053663 W
- EP 22202915